# EUROPEAN PATENT SPECIFICATION

(11) **EP 3 178 329 B1**
(45) Date of publication and mention of the grant of the patent: **14.08.2024**
(21) Application number: 15198332.7
(22) Date of filing: 08.12.2015
(51) Int. Cl.: A23C 19/032, A23C 19/076, A23C 13/16, A23C 9/123, C12R 1/46, C12R 1/225, C12N 1/20

(54) **PROCESS FOR MANUFACTURING OF QUARK OR SOUR CREAM, STARTER CULTURE, QUARK OR SOUR CREAM**
VERFAHREN ZUR HERSTELLUNG VON QUARK ODER SAURER SAHNE, STARTERKULTUR, QUARK ODER SAURE SAHNE
PROCÉDÉ DE FABRICATION DE QUARK OU DE CRÈME SURE, CULTURE DE LEVAGE, QUARK OU CRÈME SURE

(43) Date of publication of application: 14.06.2017
(73) Proprietor: DSM IP Assets B.V., 6411 TE Heerlen (NL)
(72) Inventor: AUBERT, Cécile, 6100AA ECHT (NL); PRICE, Claire Emile, 6100AA ECHT (NL)
(74) Representative: dsm-firmenich IP

(56) References cited:
- EP-A1- 0 144 274
- WO-A1-2012/136833
- WO-A1-2012/159922
- DE-A1- 3 822 082
- GB-A- 2 092 426
- IE-A1- 911 465
- CHAMPAGNE C P ET AL: "Fresh-cheesemilk formulation fermented by a combination of freeze-dried citrate-positive cultures and exopolysaccharide-producing lactobacilli with liquid lactococcal starters", FOOD RESEARCH INTERNATIONAL, ELSEVIER APPLIED SCIENCE, BARKING, GB, vol. 39, no. 6, 1 July 2006 (2006-07-01), pages 651 - 659, XP025014253, ISSN: 0963-9969, [retrieved on 20060701], DOI: 10.1016/J.FOODRES.2006.01.002
- "Handbook of food science, technology, and engineering, Volume 4", 1 January 2006, Taylor & Francis, article HUI Y: "Handbook of food science, technology, and engineering, Volume 4", pages: 177 - 6, XP093054691
- "SECOND EDITION DAIRY PROCESSING AND QUALITY ASSURANCE", 1 January 2016, WILEY BLACKWELL, article CHANDAN R.: "SECOND EDITION DAIRY PROCESSING AND QUALITY ASSURANCE", pages: 239, XP093054694
- "LIVE - Encylopedia of Membranes", 1 January 2015, SPRINGER LINK, article SCHIRALDI CHIARA ET AL: "Mesophilic Organsims", pages: 1 - 2, XP093134002

## Description

### Field of the invention

The present invention relates to a process for manufacturing fermented milk products, in particular quark. Further, the present invention relates to a starter composition. According another aspect, the present invention relates to a fermented milk product, in particular quark and / or sour cream.

### Background of the invention

Lactic acid bacteria are extensively used for production of fermented foods, and they greatly contribute to flavor, texture and overall characteristics of these products. An old and well known example is yoghurt which probably originated from the Middle East and which still makes up more than half of the fermented milk production - or approximately 19 million tons in 2008 (source: Euromonitor). Fermented milks as e.g. yoghurts are popular due to the healthy image and pleasant sensory properties.

In fermentation of milk, the lactic acid bacteria produce lactic acid due to consumption of lactose which reduces the pH and leads to the formation of a protein coagulum, i.e. curd. Further, Some lactic acid bacteria can also produce exopolysaccharide (EPS) - long chain of sugars - that are important for the texture and appearance of the end product - EPS are retaining the water into the curd and therefore increasing texture and shininess.

In fermented milk production processes, in particular quark, the curd is generally concentrated to form the fermented milk product after a desired pH is reached due to the fermentation. In industrial scale fermentation processes, wherein fermentation tanks of for example more than 50,000 liter are used, the concentration of the curd requires time since the capacity of a downstream concentrator with for example 10,000 liter per hour is smaller than the capacity of the fermentation tank. For example, time periods of 3 to 10 hours are reported for the entire concentration of the curd formed in the fermentation tank. During this time period, the curd which is present in the fermentation tank will further acidify.

It is important that during the concentration step the further acidification, or post acidification, from the lactic acid bacteria is minimal. Otherwise, during concentration, the taste and texture of the fermented milk product will change over time as result of an ongoing fermentation. For example, the first concentrated curd after reaching the desired pH of 4.6 has a pH of 4.6, whereas curd which is concentrated later may have a pH of 4.4. This is undesired in view of providing a fermented milk product having a constant quality. Furthermore, when the concentration is carried out at a too low pH it may have consequences on the yield, such as a yield loss.

Some fermented milk products, such as quark, are in general produced by mesophilic lactic acid bacteria which are active in the temperature range of 20°C - 45°C. After reaching the desired pH of 4.6, mesophilic lactic acid bacteria are difficult to inactivate by the common method of cooling since they remain active at the cooled temperature. Further, cooling (or heating) of large fermentation tanks will also require a substantial amount of time. Therefore, cooling (or heating) is usually carried out in a smaller plate heater or cooler downstream from the fermentation tank, resulting in inefficient waiting time and the problem of post acidification.

EP0144274 discloses a process for preparing a product tasting of cheese from a milk concentrate having a dry matter content corresponding to the final product to be obtained, the said concentrate comprising the total content of the protein including the albumin, of the lactose and of the mineral salts in the original milk, characterized in that this concentrate is subjected to a first stage of fermentation by means of thermophilic bacteria under temperature conditions and for a sufficient period for attaining a pH falling within the range form 4.4 to 4.8 at the end of the fermentation, after which the concentrate obtained is cooled and subjected, in a second, flavouring stage, to the action of mesophilic and/or cryophilic bacterial thereby yielding a ripened smooth concentrate.

Consequently, there is a need for an alternative cost-efficient process for manufacturing fermented milk that provides a decreased acidification between pH 4.6 and pH 4.4 and thus provides an increased time to reach a pH of 4.4.

### Definitions

As used herein the term "milk base" is the starting material, or starting substrate, for the fermentation process to provide a fermented milk product. It includes whole milk, skim milk, fat-free milk, low fat milk, full fat milk, lactose-free or lactose-reduced milk (produced by hydrolyzing the lactose by lactase enzyme to glucose and galactose, or by other methods such as nanofiltration, electro dialysis, ion exchange chromatography and centrifugation technology), concentrated milk or dry milk.

"Fat-free milk" is non-fat or skim milk product. Low-fat milk is typically defined as milk that contains from about 1% to about 2% fat. Full fat milk often contains 2% fat or more.

The term "milk" is intended to encompass milks from mammals and plant sources or mixtures thereof. Preferably, the milk is from a mammal source. Mammals sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. Soy bean milk is preferred. In addition, the term "milk" refers to not only whole milk, but also skim milk or any liquid component derived thereof.

As used in this specification, the term "fermented milk product" or "acidified dairy product" is intended to refer to products which are obtained by the multiplication of lactic acid bacteria in a milk base leading to a milk coagulum. The milk preparation used as raw material for the fermentation may be skimmed or non-skimmed milk, optionally concentrated or in the form of powder. Furthermore, this milk preparation may have been subjected to a thermal processing operation which is at least as efficient as pasteurization. The particular characteristics of the various fermented dairy products depend upon various factors, such as the composition of milk base, the incubation temperature, the lactic acid flora and/or non-lactic acid flora. Thus, fermented dairy products manufactured herein include, for instance, various types of regular yoghurt, low fat yoghurt, non fat yoghurt, kefir, dahi, ymer, buttermilk, butterfat, sour cream and sour whipped cream as well as fresh cheeses and quark.

The term "starter composition" or "starter culture" as used herein refers to a culture of one or more food-grade micro-organisms, in particular lactic acid bacteria, which are responsible for the acidification of the milk base. Starter cultures may be fresh (liquid), frozen or freeze-dried. Freeze dried cultures need to be regenerated before use. For the production of a fermented dairy product, the starter is usually added in an amount from 0.01 to 3%, preferably from 0.01 and 0.02 % by weight of the total amount of milk base.

As used herein, the term "lactic acid bacteria" (LAB) or "lactic bacteria" refers to food-grade bacteria producing lactic acid as the major metabolic end-product of carbohydrate fermentation. These bacteria are related by their common metabolic and physiological characteristics and are usually Gram positive, low-GC, acid tolerant, non-sporulating, non-respiring, rod-shaped bacilli or cocci. During the fermentation stage, the consumption of lactose by these bacteria causes the formation of lactic acid, reducing the pH and leading to the formation of a protein coagulum. These bacteria are thus responsible for the acidification of milk and for the texture of the dairy product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

The term "weakly post-acidifying" or "low post-acidifying" refers to the acidification profile of a bacterium or a bacterial culture useful in the present invention, wherein there is a reduced acidification rate or acidification speed between pH 4.6 and 4.4. Post-acidification is the production of lactic acid occurring after the end of the fermentation. The end of fermentation is generally when a desired pH of 4.6 is reached. The phenomenon of post acidification is usually controlled by the cooling of the product after fermentation. Indeed, this cooling step stops or slows down the bacterium metabolism and thus reduces the production of lactic acid. In the process of the present invention, this cooling step has been suppressed thanks to the use of bacterium cultures with weakly post-acidifying properties as a starter. These cultures are characterized by a weak production of lactic acid at fermentation temperature after the end of the fermentation step thereby providing a substantially steady pH value.

Weakly post-acidifying bacterium cultures may be selected by the follow-up of the pH of a final fermented milk base by using any method known by the skilled person. As example, a CINAC system (CINetic ACidification) may be used. In this system a pH meter is connected to a computer recorder and pH is continuously recorded as a function of time to obtain sigmoidal curves representing the acidification. During the follow-up of the pH, the milk is maintained at fermentation temperature in a thermoregulated bath.

### Detailed description of the Invention

In a first aspect the invention provides a process for the manufacture of quark or sour cream, comprising subjecting a milk base of at least 10,000 liter milk to mesophilic fermentation at a temperature in the range of 25°C - 35°C with a starter comprising a mesophilic culture and a weakly post-acidifying-thermophilic culture until a desired pH value in the range of 4.0 and 5.0 is reached,
wherein the weakly post-acidifying thermophilic culture consists of *Streptococcus thermophilus* strain CBS140557, *Streptococcus thermophilus* strain CBS140559 or *Streptococcus thermophilus* strain CBS140561.

The present inventors surprisingly found that the addition of such a thermophilic culture having weakly post acidification properties is able to reduce the acidification after reaching a pH of 4.6 at mesophilic conditions. This is surprising since thermophilic cultures are not expected to contribute to the fermentation at mesophilic conditions significantly. The present inventors found that by using the present process, the time it takes to acidify from pH 4.6 to 4.4 increases with more than 15%. Further, the present inventors found that the time to reach pH 4.6 is not reduced. This is advantageous since the present invention can than efficiently be used in existing fermentation processes, without increasing fermentation production times to reach a pH of 4.6.

The present fermented milk product is quark or sour cream. Especially the industrial production of quark relies on the concentration of curd from fermentation tanks having a capacity of more than 50,000 L. It was found by the present inventors that using the method of the present invention in industrial scale quark production provides quark of a more constant quality, since a reduced acidification between pH 4.6 and 4.4 is noticed. In other words, by providing reduced acidification, a longer time slot is available in the industrial scale process to concentrate the curd.

The present weakly post-acidifying thermophilic culture consists of at least one weakly post-acidifying thermophilic culture of *Streptococcus thermophilus. Streptococcus thermophilus* is commonly used in the product of yoghurt. Yoghurt is produced by thermophilic fermentation conditions. The present inventors found that weakly post-acidifying thermophilic cultures commonly used for the production of yoghurt have a significant contribution in providing a low post acidification in fermented milk products obtained by mesophilic fermentation, in particular quark.

In a further preferred embodiment, the present weakly post-acidifying thermophilic culture is suitable to provide a time to reach pH 4.4 after reaching a pH of 4.6 of more than 15% of a time to reach pH 4.4 after reaching a pH of 4.6 if the milk base is subjected with a starter without the present weakly post-acidifying thermophilic culture. Preferably, the present weakly post-acidifying thermophilic culture is suitable to provide a time to reach pH 4.4 after reaching a pH of 4.6 of more than 20%, more preferably more than 25%, even more preferably more than 30%, most preferably more than 35% of a time to reach pH 4.4 after reaching a pH of 4.6 if the milk base is subjected with a starter without the present weakly post-acidifying thermophilic culture.

The present weakly post-acidifying thermophilic culture consists of *Streptococcus thermophilus* strain CBS140557, *Streptococcus thermophilus* strain CBS140559 or *Streptococcus thermophilus* strain CBS140561. These deposited strains are deposited at the Centraalbureau voor Schimmel-cultures (Fungal Biodiversity Centre), Uppsalalaan 8, 3584 CT, Utrecht, The Netherlands, on September 29, 2015, under the provisions of the Budapest Treaty.

In a preferred embodiment, the present mesophilic fermentation with a starter comprising a mesophilic culture and a thermophilic culture, comprises the present thermophilic culture within an amount of 5% to 50% (wt) of the starter, preferably within an amount of 10% to 40% (wt), more preferably within an amount of 15% to 35% (wt) or even 20% to 30% (wt) of the starter. Preferably the present mesophilic fermentation with a starter comprising a mesophilic culture and a thermophilic culture, comprises the present mesophilic culture within an amount of 50% to 95% (wt) of the starter, preferably within 60% to 90% (wt), more preferably within an amount of 65% to 85% (wt) or even 70% to 80% (wt) of the starter. The present inventors found that the addition of a thermophilic culture with an amount of 5% to 50% (wt) provides a time efficient fermentation process to reach a pH of 4.6 as well as providing an increased time to reach pH 4.4 after the end of fermentation. Therefore, the present process is easy to implement in conventional methods for the production of fermented milk products.

In yet another preferred embodiment, the present mesophilic culture comprises at least one or more mesophilic culture of the genus *Lactococcus,* more preferably the strain *Lactoccus lactis subsp lactis* and /or *Lactoccus lactis subsp cremoris.*

Preferably, the present process comprises subjecting a milk base to mesophilic fermentation with a starter comprising a mesophilic culture and a weakly post-acidifying thermophilic culture until a desired pH value in the range of 4.0 and 5.0 is reached, such as a pH value in the range of 4.5 to 5.0, such as pH 4.6, 4.65, 4.7, 4.75, 4.8, 4.85, 4.9 or pH 4.95.

In a preferred embodiment the present process further comprises a step of concentrating curd after the desired pH value in the range of 4.0 and 5.0 is reached. Concentration of the curd provides quark or sour cream, and therefore, the present process preferably comprises such a concentration step to provide quark or sour cream.

The present mesophilic fermentation is at a temperature in the range of 25°C-35°C.

Given the increased time to reach a pH of 4.4 after reaching a pH of 4.6 at the end of fermentation, the present process is advantageously used in industrial scale production of fermented milk products. Therefore, the present milk base is a milk base of at least 10,000 liter milk, preferably at least 20,000 liter milk, more preferably at least 30,000 liter milk, even more preferably at least 40,000 liter milk, most preferably at least 50,000 liter milk.

Given the beneficial low post acidification profile of the present invention, the present invention relates, according to another aspect, to a starter composition, or starter culture comprising a mesophilic culture and/or a thermophilic culture, preferably a weakly post-acidifying thermophilic culture.

The present weakly post-acidifying thermophilic culture consists of at least one weakly post-acidifying thermophilic culture of *Streptococcus thermophilus. Streptococcus thermophilus* is commonly used in the product of yoghurt. Yoghurt is produced by thermophilic fermentation conditions. The present inventors found that weakly post-acidifying thermophilic cultures commonly used for the production of yoghurt have a significant contribution in providing a low post acidification in fermented milk products obtained by mesophilic fermentation, in particular quark.

In a further preferred embodiment, the present weakly post-acidifying thermophilic culture is suitable to provide a time to reach pH 4.4 after reaching a pH of 4.6 of more than 15% of a time to reach pH 4.4 after reaching a pH of 4.6 if the milk base is subjected with a starter without the weakly post-acidifying thermophilic culture. Preferably, the present weakly post-acidifying thermophilic culture is suitable to provide a time to reach pH 4.4 after reaching a pH of 4.6 of more than 20%, more preferably more than 25%, even more preferably more than 30%, most preferably more than 35% of a time to reach pH 4.4 after reaching a pH of 4.6 if the milk base is subjected with a starter without the weakly post-acidifying thermophilic culture.

The present weakly post-acidifying thermophilic culture consists of *Streptococcus thermophilus* strain CBS140557, *Streptococcus thermophilus* strain CBS140559 or *Streptococcus thermophilus* strain CBS140561.

In yet another preferred embodiment, the present mesophilic culture comprises at least one mesophilic culture of the genus *Lactococcus,* more preferably the strain *Lactoccus lactis subsp lactis* and /or *Lactoccus lactis subsp cremoris.*

Preferably, the present starter composition is in frozen or in a freeze dried form. In a preferred embodiment, the present mesophilic fermentation with a starter comprising a mesophilic culture and a thermophilic culture, comprises the present thermophilic culture within an amount of 5% to 50% (wt) of the starter, preferably within an amount of 10% to 40% (wt), more preferably within an amount of 15% to 35% (wt) or even 20% to 30% (wt) of the starter. Preferably the present mesophilic fermentation with a starter comprising a mesophilic culture and a thermophilic culture, comprises the present mesophilic culture within an amount of 50% to 95% (wt) of the starter, preferably within 60% to 90% (wt), more preferably within an amount of 65% to 85% (wt) or even 70% to 80% (wt) of the starter.

Given the beneficial low post acidification profile of the present invention, the present invention relates, according to another aspect, to a quark or sour cream, comprising a weakly post-acidifying thermophilic culture. Preferably the present thermophilic culture is a food grade thermophilic culture.

In a preferred embodiment, the present fermented dairy product, such as quark or sour cream, is packaged in a package of at least 50 gram, preferably at least 75 gram, more preferably at least 100 gram such as 250 gram or even 500 gram.

The present weakly post-acidifying thermophilic culture consists of at least one weakly post-acidifying thermophilic culture of *Streptococcus thermophilus.*

In a further preferred embodiment, the present weakly post-acidifying thermophilic culture is suitable to provide a time to reach pH 4.4 after reaching a pH of 4.6 of more than 15% of a time to reach pH 4.4 after reaching a pH of 4.6 if the milk base is subjected with a starter without the present weakly post-acidifying thermophilic culture. Preferably, the present weakly post-acidifying thermophilic culture is suitable to provide a time to reach pH 4.4 after reaching a pH of 4.6 of more than 20%, more preferably more than 25%, even more preferably more than 30%, most preferably more than 35% of a time to reach pH 4.4 after reaching a pH of 4.6 if the milk base is subjected with a starter without the weakly post-acidifying thermophilic culture.

The present weakly post-acidifying thermophilic culture consists of *Streptococcus thermophilus* strain CBS140557, *Streptococcus thermophilus* strain CBS140559 or *Streptococcus thermophilus* strain CBS140561.

In yet another preferred embodiment, the present mesophilic culture comprises at least one or more mesophilic culture of the genus *Lactococcus,* more preferably the strain *Lactoccus lactis subsp lactis* and / or *Lactoccus lactis subsp cremoris.*

Further, the present invention relates to the use of the present weakly post-acidifying thermophilic culture of *Streptococcus thermophilus* for providing a low post acidification in the production of quark or sour cream. Preferably, the present invention relates to the use of the present weakly post-acidifying thermophilic culture of *Streptococcus thermophilus* for increasing the time to reach pH 4.4 in the production of quark or sour cream, preferably in tanks of at least 20,000 liter, preferably at least 30,000 liter, more preferably at least 40,000 liter, most preferably at least 50,000 liter.

The invention is further illustrated in the following non limiting examples.

### EXAMPLES

### Cultures

**Table 1**

| **Culture** | **Strain** | **availability** |
|---|---|---|
| DELVO^{®}TEC LL-50F (LL-50F) | *Lactococcus lactis subsp lactis* and *Lactococcus lactis subsp cremoris* | commercially available from DSM Food Specialties B.V., Delft, The Netherlands |
| DELVO^{®}TEC LL-50X (LL-50X) | *Lactococcus lactis subsp lactis* and *Lactococcus lactis subsp cremoris* | commercially available from DSM Food Specialties B.V., Delft, The Netherlands |
| DELVO^{®}ADD 200M (200M) | *Lactococcus lactis subsp cremoris* | commercially available from DSM Food Specialties B.V., Delft, The Netherlands |
| CBS 140557 | *Streptococcus thermophilus* | deposited* |
| CBS 140559 | *Streptococcus thermophilus* | deposited* |
| DELVO^{®}FRESH FC-214 (FC-214) | *Lactococcus lactis subsp lactis* and *Lactococcus lactis subsp cremoris* | commercially available from DSM Food Specialties B.V., Delft, The Netherlands |
| CBS 140561 | *Streptococcus thermophilus* | deposited* |

| | | |
|---|---|---|
| *deposited at the Centraalbureau voor Schimmel-cultures (Fungal Biodiversity Centre), Uppsalalaan 8, 3584 CT, Utrecht, The Netherlands, The Netherlands, on September 29, 2015, under the provisions of the Budapest Treaty. | | |

### Example 1

Dutch skimmed milk was inoculated with *Lactococcus lactis* strains and with *Lactococcus lactis* strains supplemented with a *Streptococcus thermophilus* strain, with a total concentration of 1 U / 1000L milk, as shown in table 1 below. The milk was inoculated at a temperature of 28°C or 32°C until a pH of 4.6 was reached. The pH of the fermented milk was measured during and after fermentation by a CINAC system (CINetic ACidification). The time to reach (TTR) pH 4.6 and pH 4.4 was measured. The results are shown in table 1 below.

**Table 2**

| **Culture (in U/1000L)** | **Strain** | **Fermentation temperature** | **TTR pH 4.6 (min)** | **TTR4.6 - TTRpH4.4 (min)** |
|---|---|---|---|---|
| 0.35U LL-50F + 0.35U LL- 50X + 0.3U 200M | *Lactococcus lactis* | 28°C | 705 | 237 |
| 0.35U LL-50F + 0.35U LL-50X + 0.3U CBS140557 | *Lactococcus lactis* + *Streptococcus thermophilus* | 28°C | 704 | 314 |
| 0.35U LL-50F + 0.35U LL-50X + 0.3U CBS140559 | *Lactococcus lactis* + *Streptococcus thermophilus* | 28°C | 672 | 321 |
| 0.5U LL-50F + 0.5U LL-50X | *Lactococcus lactis* | 28°C | 662 | 244 |
| 0.35U LL-50F + 0.35U LL-50X + 0.3U 200M | *Lactococcus lactis* | 32°C | 515 | 133 |
| 0.35U LL-50F + 0.35U LL-50X + 0.3U CBS140557 | *Lactococcus lactis* + *Streptococcus thermophilus* | 32°C | 526 | 188 |
| 0.35U LL-50F + 0.35U LL-50X + 0.3U CBS140559 | *Lactococcus lactis* + *Streptococcus thermophilus* | 32°C | 520 | 168 |
| 0.5U LL-50F + 0.5U LL-50X | *Lactococcus lactis* | 32°C | 516 | 145 |

Table 2 above clearly shows that the combination of a mesophilic *Lactococcus lactis* culture with a *Streptococcus thermophilus* provides an increased time to reach a pH of 4.4. Further, table 2 shows that the time to reach a pH of 4.6 is not significantly changed, thus the addition of the *Streptococcus thermophilus* does not provide an undesired increase of the time to reach pH 4.6. This increased time to reach a pH of 4.4 provides more time for separation of curd from the fermented skimmed milk to produce quark.

### Example 2

Sour cream was made by inoculating cream (15% fat) with strain FC-214 at 111/ 1000L milk and with strain 500F in a concentration of 0.4U per 1000L milk or 1U per 1000L milk. The milk was inoculated at a temperature of 28°C until a pH of 4.6 was reached. The pH of the sour cream was measured during and after fermentation and after 20 hours storage by a CINAC system (CINetic ACidification). The results are shown in table 3 below.

**Table 3**

| **Culture (in U/1000L)** | **Strains** | **TTR pH 4.6 (min)** | **TTR4.6 - TTRpH4.4 (min)** | **pH after 20hr** |
|---|---|---|---|---|
| FC-214 (1U) + CBS140561 (1U) | *Lactococcus lactis* + *Streptococcus thermophilus* | 476 | 256 | 4.3 |
| FC-214 (1U) + CBS140561 (0.4U) | *Lactococcus lactis* + *Streptococcus thermophilus* | 486 | 273 | 4.3 |
| FC-214 (1U) | *Lactococcus lactis* | 454 | 191 | 4.2 |

Table 3 above clearly shows that the time to decrease from pH 4.6 to pH 4.4 is increased with 60 to 80 minutes by using a mesophilic culture with added *Streptococcus thermophilus.* Further, table 3 above shows that the end pH after 20 hr storage at 6°C is increased by 0,05UpH.

## Claims

1. A process for the manufacture of quark or sour cream, comprising subjecting a milk base of at least 10,000 liter milk to mesophilic fermentation at a temperature in the range of 25°C - 35°C with a starter comprising a mesophilic culture and a weakly post-acidifying thermophilic culture until a desired pH value in the range of 4.0 and 5.0 is reached,
wherein the weakly post-acidifying thermophilic culture consists of *Streptococcus thermophilus* strain CBS140557, *Streptococcus thermophilus* strain CBS140559 or *Streptococcus thermophilus* strain CBS140561.

2. Process according to any of the preceding claims, wherein the weakly post-acidifying thermophilic culture is suitable to provide a time to reach pH 4.4 after reaching a pH of 4.6 of more than 15% of a time to reach pH 4.4 after reaching a pH of 4.6 if the milk base is subjected with a starter without the weakly post-acidifying thermophilic culture.

3. Process according to any of the preceding claims, wherein the mesophilic culture comprises at least one mesophilic culture of the genus *Lactococcus,* more preferably the strain *Lactoccus lactis subsp lactis* and / or *Lactoccus lactis subsp cremoris.*

4. Process according to any of the preceding claims, further comprising a step of concentrating curd after the desired pH value in the range of 4.0 and 5.0 is reached.

5. Process according to any of the preceding claims, wherein the mesophilic fermentation with a starter comprising a mesophilic culture and a thermophilic culture, comprises the thermophilic culture within an amount of 5% to 50% (wt) of the starter, and comprises the mesophilic culture within an amount of 50% to 95% (wt) of the starter.

6. A starter composition comprising a mesophilic culture and a weakly post-acidifying thermophilic culture, wherein the weakly post-acidifying thermophilic culture consists of *Streptococcus thermophilus* strain CBS140557, *Streptococcus thermophilus* strain CBS140559 or *Streptococcus thermophilus* strain CBS140561.

7. A starter composition according to claim 6, wherein the weakly post-acidifying thermophilic culture is suitable to provide a time to reach pH 4.4 after reaching a pH of 4.6 of more than 15% of a time to reach pH 4.4 after reaching a pH of 4.6 if a milk base is subjected with a starter without the weakly post-acidifying thermophilic culture.

8. A starter composition according to any one of claims 6 to 7, wherein the mesophilic culture comprises at least one mesophilic culture of the genus *Lactococcus,* more preferably the strain *Lactoccus lactis subsp lactis* and / or *Lactoccus lactis subsp* cremoris,preferably, the starter composition is in frozen or in a freeze dried form.

9. A starter composition according to any one of claims 6 to 8, comprising the thermophilic culture within an amount of 5% to 50% (wt) of the starter, and comprising the mesophilic culture within an amount of 50% to 95% (wt) of the starter.

10. Quark or sour cream comprising a weakly post-acidifying thermophilic culture consisting of *Streptococcus thermophilus* strain CBS140557, *Streptococcus thermophilus* strain CBS140559 or *Streptococcus thermophilus* strain CBS140561.

## Patentansprüche

1. Verfahren zur Herstellung von Quark oder Sauerrahm, umfassend Durchführen einer mesophilen Fermentation von wenigstens 10.000 Liter Milch als Milchgrundstoff bei einer Temperatur im Bereich von 25°C - 35°C mit einem eine mesophile Kultur und eine schwach nachsäuernde thermophile Kultur umfassenden Starter, bis ein gewünschter pH-Wert im Bereich von 4,0 und 5,0 erreicht ist,
wobei die schwach nachsäuernde thermophile Kultur aus *Streptococcus thermophilus-Stamm* CBS140557, *Streptococcus thermophilus-Stamm* CBS140559 oder *Streptococcus thermophilus-Stamm* CBS140561 besteht.

2. Verfahren nach einem der vorhergehenden Ansprüche, wobei die schwach nachsäuernde thermophile Kultur geeignet ist, eine Zeit zum Erreichen von pH 4,4 nach Erreichen eines pH von 4,6 zu liefern, die mehr als 15 % einer Zeit zum Erreichen von pH 4,4 nach Erreichen eines pH von 4,6, wenn der Milchgrundstoff mit einem Starter ohne die schwach nachsäuernde thermophile Kultur in Kontakt gebracht wird, beträgt.

3. Verfahren nach einem der vorhergehenden Ansprüche, wobei die mesophile Kultur wenigstens eine mesophile Kultur der Gattung *Lactococcus,* bevorzugter den Stamm *Lactococcus lactis subsp lactis* und / oder *Lactoccus lactis subsp cremoris* umfasst.

4. Verfahren nach einem der vorhergehenden Ansprüche, ferner umfassend einen Schritt, bei dem Bruch konzentriert wird, nachdem der gewünschte pH-Wert im Bereich von 4,0 und 5,0 erreicht ist.

5. Verfahren nach einem der vorhergehenden Ansprüche, wobei die mesophile Fermentation mit einem eine mesophile Kultur und eine thermophile Kultur umfassenden Starter die thermophile Kultur in einer Menge von 5 Gew.-% bis 50 Gew.-% des Starters und die mesophile Kultur in einer Menge von 50 Gew.-% bis 95 Gew.-% des Starters umfasst.

6. Starterzusammensetzung, umfassend eine mesophile Kultur und eine schwach nachsäuernde thermophile Kultur, wobei die schwach nachsäuernde thermophile Kultur aus *Streptococcus thermophilus-Stamm* CBS140557, *Streptococcus thermophilus-Stamm* CBS140559 oder *Streptococcus thermophilus-Stamm* CBS140561 besteht.

7. Starterzusammensetzung nach Anspruch 6, wobei die schwach nachsäuernde thermophile Kultur geeignet ist, eine Zeit zum Erreichen von pH 4,4 nach Erreichen eines pH von 4,6 zu liefern, die mehr als 15 % einer Zeit zum Erreichen von pH 4,4 nach Erreichen eines pH von 4,6, wenn der Milchgrundstoff mit einem Starter ohne die schwach nachsäuernde thermophile Kultur in Kontakt gebracht wird, beträgt.

8. Starterzusammensetzung nach einem der Ansprüche 6 bis 7, wobei die mesophile Kultur wenigstens eine mesophile Kultur der Gattung *Lactococcus,* bevorzugter den Stamm *Lactococcus lactis subsp lactis* und / oder *Lactoccus lactis subsp cremoris* umfasst, bevorzugt die Starterzusammensetzung in gefrorener oder in einer gefriergetrockneten Form vorliegt.

9. Starterzusammensetzung nach einem der Ansprüche 6 bis 8, umfassend die thermophile Kultur in einer Menge von 5 Gew.-% bis 50 Gew.-% des Starters und die mesophile Kultur in einer Menge von 50 Gew.-% bis 95 Gew.-% des Starters.

10. Quark oder Sauerrahm, umfassend eine schwach nachsäuernde thermophile Kultur, die aus *Streptococcus thermophilus-Stamm* CBS140557, *Streptococcus thermophilus-Stamm* CBS140559 oder *Streptococcus thermophilus-Stamm* CBS140561 besteht.

## Revendications

1. Procédé pour la fabrication de fromage blanc ou de crème fraîche, comprenant la soumission d'une base de lait d'au moins 10 000 litres de lait à une fermentation mésophile à une température dans la plage de 25 °C à 35 °C avec un starter comprenant une culture mésophile et une culture thermophile faiblement post-acidifiante jusqu'à ce qu'une valeur de pH souhaitée dans la plage de 4,0 à 5,0 soit atteinte,
la culture thermophile faiblement post-acidifiante étant constituée de la souche CBS140557 de *Streptococcus thermophilus,* de la souche CBS140559 de *Streptococcus thermophilus* ou de la souche CBS140561 de *Streptococcus thermophilus.*

2. Procédé selon l'une quelconque des revendications précédentes, la culture thermophile faiblement post-acidifiante étant appropriée pour fournir une durée pour atteindre un pH 4,4 après avoir atteint un pH de 4,6 de plus de 15 % d'une durée pour atteindre un pH 4,4 après avoir atteint un pH de 4,6 si la base de lait est soumise avec un starter sans la culture thermophile faiblement post-acidifiante.

3. Procédé selon l'une quelconque des revendications précédentes, la culture mésophile comprenant au moins une culture mésophile du genre *Lactococcus,* plus préférablement la souche *Lactococcus lactis subsp lactis* et/ou *Lactoccus lactis subsp cremoris.*

4. Procédé selon l'une quelconque des revendications précédentes, comprenant en outre une étape de concentration de caillé après que la valeur de pH souhaitée dans la plage de 4,0 à 5,0 est atteinte.

5. Procédé selon l'une quelconque des revendications précédentes, la fermentation mésophile avec un starter comprenant une culture mésophile et une culture thermophile, comprenant la culture thermophile en une quantité de 5 % à 50 % (en poids) du starter, et comprenant la culture mésophile en une quantité de 50 % à 95 % (en poids) du starter.

6. Composition de starter comprenant une culture mésophile et une culture thermophile faiblement post-acidifiante, la culture thermophile faiblement post-acidifiante étant constituée de la souche CBS140557 de *Streptococcus thermophilus,* de la souche CBS140559 de *Streptococcus thermophilus* ou de la souche CBS140561 de *Streptococcus thermophilus.*

7. Composition de starter selon la revendication 6, la culture thermophile faiblement post-acidifiante étant appropriée pour fournir une durée pour atteindre un pH 4,4 après avoir atteint un pH de 4,6 de plus de 15 % d'une durée pour atteindre un pH 4,4 après avoir atteint un pH de 4,6 si la base de lait est soumise avec un starter sans la culture thermophile faiblement post-acidifiante.

8. Composition de starter selon l'une quelconque des revendications 6 à 7, la culture mésophile comprenant au moins une culture mésophile du genre *Lactococcus,* plus préférablement la souche *Lactococcus lactis subsp lactis* et/ou *Lactoccus lactis subsp cremoris,* préférablement la composition de starter étant sous forme congelée ou sous une forme lyophilisée.

9. Composition de starter selon l'une quelconque des revendications 6 à 8, comprenant la culture thermophile en une quantité de 5 % à 50 % (en poids) du starter, et comprenant la culture mésophile en une quantité de 50 % à 95 % (en poids) du starter.

10. Fromage blanc ou crème fraîche comprenant une culture thermophile faiblement post-acidifiante, la culture thermophile faiblement post-acidifiante étant constituée de la souche CBS140557 de *Streptococcus thermophilus,* de la souche CBS140559 de *Streptococcus thermophilus* ou de la souche CBS140561 de *Streptococcus thermophilus.*
